# EUROPÄISCHE PATENTSCHRIFT

(11) **EP 0 626 180 B1**
(45) Veröffentlichungstag und Bekanntmachung des Hinweises auf die Patenterteilung: **04.08.1999**
(21) Anmeldenummer: 93108667.2
(22) Anmeldetag: 28.05.1993
(51) Int. Cl.: A61M 16/00, F16K 15/16

(54) **Mundstück für Inhalationstherapiegeräte**
Mouthpiece for inhalation therapy apparatus
Embout buccal pour appareil de thérapie par inhalation

(43) Veröffentlichungstag der Anmeldung: 30.11.1994
(73) Patentinhaber: PARI GmbH Spezialisten für effektive Inhalation, 82319 Starnberg (DE)
(72) Erfinder: Brugger, Stefan, D-82301 Starnberg (DE); Knoch, Martin, Dr., D-82335 Berg (DE)
(74) Vertreter: Ritter und Edler von Fischern, Bernhard,Dipl.-Ing.

(56) Entgegenhaltungen:
- EP-A- 0 231 955
- WO-A-86/01731
- US-A- 2 470 297

## Beschreibung

Die vorliegende Erfindung betrifft ein Mundstück für Inhalationstherapiegeräte.

Ein Inhalationstherapiegerät, bei dem das erfindungsgemäße Mundstück einqesetzt werden kann, ist beispielsweise aus EP-A-0 281 650 bekannt. Das Gerät besitzt einen Vernebler mit Zuluftkamin und Verneblerraum, in dem eine Zerstäuberdüse ein medikamenthaltiges; Aerosol erzeugt. Das Aerosol besteht aus einem feinen Tröpfchen- oder Partikelnebel, der vom Patienten eingeatment wird. Das bekannte Inhalationstherapiegerät besitzt dazu ein Mundstück, das auf einen Auslaßstutzen des Verneblers aufgesteckt ist und durch das der Patient das Aerosol einatmet. Dabei strömt Umgebungsluft durch den Zuluftkamin und den Verneblungsraum in das Mundstück nach. Beim Ausatmen in das Mundstück hinein wird ein im Zuluftkamin des Zerstäubers vorgesehenes Rückschlagventil geschlossen. Um dennoch ein Ausatmen in das Mundstück hinein zu ermöglichen, ist an dem bekannten Mundstück ein sich während des Ausatmungsvorgangs öffnendes Rückschlagventil vorgesehen, das in einem abgezweigten rohrförmigen Stutzen angeordnet ist und durch das die Luft in die Umgebung abgeführt wird.

Aus WO 86/01731 ist ein Mundstück für Inhalationstherapiegeräte bekannt, das einen im wesentlichen rohrförmigen Grundkörper, in dessen Wandung eine Öffnung angeordnet ist, und ein Rückschlagventil besitzt, das in dieser Öffnung vorgesehen ist. Das Rückschlagventil ist realisiert in Form eines Ventileinsatzes, bestehend aus einem zylindrischen Einsteckkörper und einem flachen elastischen Element, das an einer über Speichen am zylindrischen Einsteckkörper befestigten Nabe gehaltert ist. Der Wandabschnitt des Mundstücks, in dem sich die Öffnung befindet, ist im wesentlichen eben. Jedoch ist das Ventilelement nicht unmittelbar zum Verschließen der Öffnung in der Mundstückwand geeignet und vorgesehen, so daß aus diesem Grund die Abmessung und die Form des Ventilelements nicht an die Öffnung in der Mundstückwand angepaßt sind. Ferner ist das Ventilelement nicht am ebenen Wandabschnitt des Mundstücks unmittelbar befestigt, sondern an dem Einsteckkörper. Aufgrund der Anordnung des Rückschlagventils in einem zylindrischen Einsteckkörper wird im Bereich der Öffnung des Mundstücks ein Totvolumen erzeugt, in dem sich die Aerosolpartikel niederschlagen und ansammeln können. Dies ist insbesondere im Hinblick auf die sehr kleinen Partikelgrößen, die für therapeutische Inhalationsgeräte erforderlich sind, nachteilig.

Ein an der Wand eines Inhalationsgerätes unmittelbar befestigtes Ventilelement ist bekannt aus US-A-2.470.297. Das Ventilelement, das in der Gebrauchslage des Inhalationsgerätes an der Unterseite angeordnet ist, gibt eine Öffnung, die sich in einem Mundstückbereich des Inhalationsgerätes befindet, frei, wenn der Patient in das Inhalationsgerät hinein ausatmet. Wenn der Patient durch das Inhalationsgerät einatmet, legt sich das Ventilelement auf die Öffnung und verschließt diese. Die Anordnung des Ventilelements auf der Unterseite des Inhalationsgerätes ist nachteilig, da aufgrund des Eigengewichts das Ventilelement die Öffnung freigibt. Um das Zuströmen von Atemluft durch die Öffnung zu vermeiden, muß das Ventilelement erst angehoben werden, so daß es sich auf die Außenwand des Inhalationsgerätes legt. Auch bringt die Anordnung der Öffnung und des Ventilelements auf der Unterseite des Inhalationsgeräts die Gefahr mit sich, daß sich in dem in der Öffnung entstehenden Totvolumen das vom Patienten einzuatmende Medikament ansammelt.

Es ist daher die Aufgabe der vorliegenden Erfindung, ein Mundstück für Inhalationstherapiegeräte zu schaffen, bei dem das Ruckschlagventil so gestaltet ist, daß die Strömung des Aerosols während des Einatmungsvorgangs weitestgehend unbeeinflußt bleibt und insbesondere einem Abscheiden des Aerosols weiter entgegengewirkt wird.

Die erfindungsgemäße Aufgabe wird gelöst durch ein Mundstück eines Inhalationstherapiegeräts mit einem Grundkörper mit im wesentlichen rohrförmiger Gestalt, der zumindest einen im wesentlichen ebenen Wandabschnitt aufweist, in dem eine Öffnung mit einer umlaufenden Randstufe, die sich in die Öffnung hinein erstreckt, ausgebildet ist, und einem Rückschlagventil zum Abdichten der Öffnung während eines Einatmungsvorgangs durch das Mundstück und zum Abführen von Atemluft durch die Öffnung während eines in das Mundstück gerichteten Ausatmungsvorgangs, das ein flaches, elastisches Ventilelement umfaßt, das an dem Grundkörper in dem Bereich des ebenen Wandabschnitts derart befestigt ist, daß es in einer Ruhestellung aufgrund der wirkenden elastischen Kräfte von außen am Grundkörper anliegt und die Öffnung verschließt, und daß es zur Freigabe der Öffnung von dem Grundkörper weg teilweise abhebbar ist, dessen Abmessungen und Form zum Verschließen der Öffnung an diese angepaßt sind, und das auf der Randstufe in der Ruhestellung derart aufliegt, daß das flache, elastische Ventilelement im wesentlichen in der Öffnung angeordnet ist.

Indem erfindungsgemäß das flache elastische Ventilelement auf der Randstufe der Öffnung in der Mundstückwand aufliegt, wird nicht nur erreicht, daß das zur Verfügung stehende Totvolumen für die Abscheidung des Aerosols reduziert wird und die Beeinflussung des Aerosols damit kleiner wird, sondern auch daß das flache Ventilelement gegen unbeabsichtigte Berührung oder Beschädigung besser geschützt ist. Die erfindungsgemäße Anordnung des flachen Ventilelements erreicht damit nicht nur die Lösung der zuvor genannten Aufgabe, sondern auch einen weiteren Vorteil durch eine konstruktive Maßnahme.

In einer vorteilhaften Ausgestaltung weist der Grundkörper einen Anschlußbereich für den Anschluß des Mundstücks an das Inhalationstherapiegerät, einen ergonomisch geformten Bereich zur Aufnahme in den Mund und einen Übergangsbereich zur Überführung der Form des Anschlußbereichs in die Form des ergonomisch geformten Bereichs auf, und ist der im wesentlichen ebene Wandabschnitt, in dem die Öffnung ausgebildet ist, im Übergangsbereich angeordnet.

Weiter kann das flache elastische Ventilelement mittels einer Klemmeinrichtung außen am Grundkörper gehalten sein.

In einer weiteren Ausgestaltung ist an die Klemmeinrichtung einstöckig eine Ventilelementabdeckung ungeformt, die zum Schutz des flachen., elastischen Ventilelements außen am Grundkörper über dem Ventilelement angeordnet ist.

Das erfindungsgemäße Mundstück kann schließlich so ausgebildet sein, daß das flache elastische Ventilelement eine Arretieröffnung aufweist, daß der Grundkörper eine Arretieröffnung aufweist, und daß die Klemmeinrichtung aus einer geöffneten Stellung in eine das Ventilelement einklemmende Stellung klappbar ist und einen Arretiervorsprung aufweist, der in der das Ventilelement einklemmenden Stellung der Klemmeinrichtung in die zueinander ausgerichteten Arretieröffnungen des Ventilelements und des Grundkörpers eingreift.

Das flache, elastische Ventilelement ist aus einem Kunststoff oder Gummi hergestellt.

Erfindungsgemäß wird die Wand des Mundstückgrundkörpers im Bereich der Öffnung durch ein flaches, elastisches Ventilelement nachgebildet. Die innere Oberfläche des Mundstückgrundkörpers wird quasi durch die zum Innenraum des Grundkörpers gerichtete Oberfläche des flachen, elastischen Ventilelements im Bereich der Öffnung ersetzt. Dadurch wird erreicht, daß der im Bereich des Ventils auftretende Totraum deutlich verkleinert ist und aufgrund der Nachbildung der Innenwand praktisch keine Beeinflussung der Strömung im Mundstück auftritt.

Im folgenden wird die Erfindung anhand von

Ausführungsbeispielen beschrieben, die in den Zeichnungen gezeigt sind. Im Einzelnen zeigt:
Fig. 1 ein Beispiel eines Mundstücks an einem beispielhaft dargestellten Vernebler im Querschnitt;
Fig. 2 ein weiteres Beispiel eines Mundstücks im Querschnitt;
Fig. 3 eine perspektivische Ansicht eines Ausführungsbeispiels des erfindungsgemäßen Mundstücks, und
Fig. 4 eine vergrößerte Ansicht eines Details des Ausführungsbeispiels.

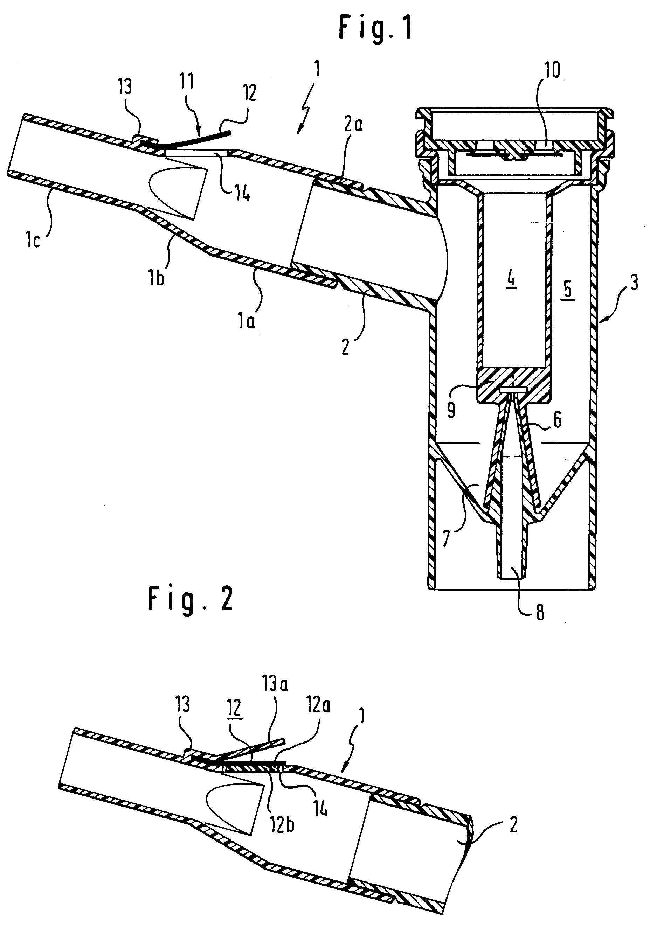

Unter Bezugnahme auf Fig. 1 wird im folgenden zunächst grundlegend der Aufbau und die Funktion eines Mundstücks 1 erläutert, das in der in Fig. 1 gewählten Darstellung auf einen Ansaugstutzen 2 eines Verneblers 3 aufgesteckt ist. Selbstverständlich kann ein derartiges Mundstück im Zusammenhang mit anderen Inhalationstherapiegeräten verwendet werden, die ein Aerosol zur Einatmung für einen Patienten bereitstellen.

Der beispielhaft gewählte Vernebler 3 besitzt einen Zuluftkamin 4, durch den beim Einatmen Umgebungsluft in die Verneblungskammer 5 eingesaugt wird. In der Verneblungskammer erzeugt eine Düse 6 ein Aerosol aus einem pulverförmigen oder flüssigen Zerstäubungsgut, insbesondere einem Medikament, das am unteren Ende der Düse 6 in einem Zerstäubungsgutbehälter 7 bevorratet wird. Zur Aerosolerzeugung wird der Düse 6 durch einen Druckluftkarial 8 Druckluft zugeführt, die am oberen Ende der Düse austritt und durch zwei Zerstäubungsgutkanäle, die benachbart zur Austrittsöffnung des Druckluftkanals angeordnet sind, das Zerstäubungsgut ansaugt. Unterstützt durch ein Luftstromsteuer 9 mit Prallkante wird durch Verneblung ein Aerosol erzeugt, das im Verneblungsraum 5 bereitgehalten wird. Die Aerosolerzeugung kann kontinuierlich oder jeweils kurz vor oder während eines Einatmungsvorgangs erzeugt werden. Für die kontinuierliche Aerosolerzeugung wird ein kontinuierlicher Druckluftstrom durch den Druckgaskanal 8 der Düse 6 zugeführt. In diesem Fall ist der Zuluftkamin 4 nach außen durch ein Rückschlagventil 10 abgeschlossen, das verhindert, daß das im Verneblungsraum 5 erzeugte Aerosol sich durch den Zuluftkamin 4 nach außen verflüchtigt.

Beim Einatmen saugt der Patient das im Verneblungsraum 5 vorgehaltene Aerosol durch das Mundstück 1 und den Ansaugstutzen 2 ein. Durch den erzeugten Unterdruck strömt Umgebungsluft durch das sich öffnende Rückschlagventil 10 über den Zuluftkamin 4 in den Verneblungsraum 5 nach und wird von dort durch das Mundstück 1 und den Ansaugstutzen 2 angesaugt. Bei kontinuierlicher Aerosolerzeugung vermischt sich die Umgebungsluft fortlaufend mit dem erzeugten Aerosol. Die Strömungsquerschnitte des Zuluftkamins 4, des Verneblungsraums E') und des Ansaugstutzens 2 sowie das Verhalten des Rückschlagventils 10 sind so auf die Zerstäubungsleistung der Düse 6 abgestimmt, daß ein Aerosol mit gewünschter Konzentration und Partikelverteilung während des Einatmungsvorgangs vom Patienten eingeatmet werden kann.

Im Bereich des Mundstücks 1 und des Ansaugstutzens 2 soll keine weitere Umgebungsluft dem angesaugten Aerosol zugeführt werden, um mit Hilfe des Zuluftstroms eine maximale Ausbringung des Zerstäubungsguts zu gewährleisten. Darüber hinaus wird eine strömungsgünstige Geometrie im Inneren des Mundstücks angestrebt, um ungewünschte Verwirbelungen und das Entstehen von Inhomogenitäten im Aerosol zu vermeiden. Für den Einatmungsvorgang besitzt ein ideales Mundstück deshalb die Form eines geschlossenen, im wesentlichen rohrförmigen Grundkörpers, bei dem sich die Querschnitte und Querschnittsformen kontinuierlich ändern. Dabei bleibt die Querschnittsfläche gleich oder verkleinert sich in Richtung der Strömung beim Einatmen. Die Strömung wird bei einer derartigen Ausgestaltung nicht verwirbelt und das Niederschlagen der Aerosoltröpfchen bzw. -partikel wird minimiert.

Beim Ausatmen darf jedoch die ausgeatmete Luft nicht auf umgekehrtem Weg wieder in die Umgebung freigegeben werden, da ein Ausatmen durch. den Ansaugstutzen 2, den Verneblungsraum 5 und den Zuluftkamin 4 dazu führen wurde, daß das kontinuierlich erzeugte Aerosol oder zumindest Aerosolreste aus dem Verneblungsraum 5 herausbefördert werden, wodurch das in Form des Aerosols vorliegende Zerstäubungsgut verlorengeht. Aus diesem Grund ist das Rückschlagventil 10 vorgesehen, das den Zuluftkamin beim Ausatmen verschließt, so daß ein Ausatmen durch den Zuluftkamin 4 und damit durch den Verneblungsraum 5 nicht möglich ist.

Um den Patienten aber ein Ausatmen in das Mundstück hinein zu ermöglichen, ist das Mundstück 1 mit einem Ventil 11 ausgestattet. Dieses Ventil ist so ausgebildet, daß es die Wandung eines idealen Mundstücks so weit wie möglich nachbildet. Der Grundgedanke liegt darin, ein Mundstück mit einem Ventil derart auszustatten, daß die Wandung des Mundstücks an der Stelle, an der sich die Öffnung des Ventils befindet, durch ein elastisches Ventilelement so nachgebildet wird, daß die Abweichung von der ursprünglich vorhandenen Wand so gering wie möglich ist. Dadurch wird bereits eine geringe Beeinflussung der Strömungsverhältnisse erreicht und das Ablagern von Aerosoltröpfchen oder -partikeln reduziert.

In diesem Sinne ist das Mundstück 1 in drei Abschnitte 1a, 1b und 1c unterteilt. Das Ventil 11 ist im Bereich 1b angeordnet, der einen Übergang zwischen dem Bereich 1a und 1c schafft. Der Bereich la des Mundstücks 1 ist im wesentlichen kreiszylindrisch und an den rohrförmigen Ansaugstutzen 2 des Inhalationstherapiegeräts angepaßt. Dadurch kann das Mundstück 1 auf den Ansaugstutzen 2 derart aufgesteckt werden, daß zum einen ein sicherer Sitz gewährleistet wird und zum anderen ein unerwünschtes Zuströmen von Umgebungsluft aufgrund der erreichbaren Dichtigkeit an der Aufsteckstelle 2a vermieden wird. Im Bereich 1c ist das Mundstück aus ergonomischen Gründen abgeflacht und besitzt beispielsweise einen im wesentlichen elliptischen Querschnitt. Zwischen den beiden Bereichen 1a und 1c liegt der Bereich 1b, der die eine Form in die andere Strömunqsqünstig überführt. Das Mundstück 1 besitzt im Bereich 1b zumindest eine im wesentlichen ebene Wandfläche, in der die Öffnung 14 für die Atemluft während des Ausatmungsvorgangs ausgebildet ist und in deren unmittelbaren Bereich das Ventil 11 angeordnet ist.

Bei dem in Fig. 1 gezeigten Mundstück umfaßt das Ventil 11 ein flaches elastisches Element 12, das an einem Ende mit Hilfe einer Klemmeinrichtung 13 außen am Mundstück 1 befestigt ist. Dar, freie Ende des elastischen Elements 12 liegt in einer Ruhestellung flach auf der ebenen Wandfläche des Bereichs 1b auf und verschließt die in diesem Bereich vorgesehene Öffnung 14. Dazu besitzt das elastische Element 12 entsprechende Abmessungen und eine an die Öffnung 14 angepaßte Form. Die Klemmeinrichtung 13 ist nicht im Bereich der ebenen Wandfläche angeordnet, sondern derart in deren Nähe, daß das Ventilelement 12 gehalten wird, das sich dazu bis zur Klemmeinrichtung erstreckt.

In Fig. 1 ist das elastische Element 12 in einer angehobenen Stellung dargestellt, die sich ergibt, wenn der Patient in das Mundstück 1 hinein ausatmet. Beim Einatmen durch das Mundstück 1 hindurch unterstützt der im Mundstück entstehende Unterdruck die elastischen Kräfte, die in der Ruhestellung dafür sorgen, daß die Öffnung 14 des Mundstücks von dem elastischen Ventilelement 12 verschlossen wird. Somit kann während des Einatmens keine Umgebungsluft durch die Öffnung 14 in das Mundstück und damit in das angesaugte Aerosol gelangen.

Das elastische Element 12 ist an dem ebenen Wandstück des Bereichs 1b des Mundstücks so angeordnet, daß das im Bereich der Öffnung 14 fehlende Wandstück des Mundstücks 1 durch das elastische Element 12 ersetzt wird. Lediglich das Volumen, das sich aufgrund der Öffnungsfläche der Öffnung 14 und der Wanddicke des Mundstücks 1 ergibt, beeinflußt bei dem Ventil 11 die Strömungsverhältnisse im Mundstück 1. Jedoch ist durch die Ausgestaltung des Mundstückventils 11 dieses Volumen bereits bei dieser Ausführungsform klein, so daß die Strömungsverhältnisse fast den Strömungsverhältnissen entsprechen, die mit einem Mundstück ohne Öffnung erreicht werden. Das Volumen im Bereich der Öffnung 14 ist so klein, daß nahezu keine Abscheidung der im Aerosol enthaltenen Tröpfchen oder Partikel stattfindet.

Die Strömung wird durch den Totraum im Bereich des Ventils kaum beeinflußt.

In Fig. 2 ist ein weiteres Mundstück 1 für ein Inhalationstherapiegerät gezeigt, von dem aber nur ein Teil des Ansaugstutzens 2 dargestellt ist. Bei diesem Mundstück ist das elastische Ventilelement 12 in der Ruhestellung gezeigt, in der es die Öffnung 14 abdeckt. Aus der Darstellung der Fig. 2 geht hervor, daß der Totraum, der durch die Öffnung 14 und das sich in Ruhestellung befindende elastische Ventilelement 12 festgelegt wird, nahezu vollständig beseitigt ist. Dazu ist das elastische Ventilelement 12 zweischichtig aufgebaut. Die erste Schicht 12a, die mittels der ihr innewohnenden elastischen Kräfte bewirkt, daß das Ventilelement 12 in der Ruhestellung am Grundkörper 1 anliegt, entspricht im wesentlichen dem elastischen Ventilelement 12 des zuvor erläuterten Mundstücks. Darüber hinaus weist bei dem Mundstück gem. Fig. 2 das Ventilelement 12 eine zweite Schicht 12b auf, die an der der Öffnung 14 zugewandten Seite der ersten Schicht 12a angeordnet ist und deren Form und Abmessungen so an die Öffnung 14 angepaßt sind, daß dieser Teil des Ventilelements in der Öffnung angeordnet werden kann und daß dennoch eine Bewegung des Ventilelements 12 möglich ist, durch die die Öffnung 14 während des Ausatmungsvorgangs freigegeben wird. Die Dicke der Schicht 12b des Ventilelements 12 entspricht der Wanddicke des Grundkörpers 1 des Mundstücks. In der Ruhestellung wird daher die Öffnung 14 im Grundkörper 1 so verschlossen, daß die Innenwand des Grundkörpers 1 nahezu unterbrechungsfrei fortgesetzt wird, indem die nach innen gewandte Oberfläche der zweiten Schicht 12b die Innenwand des Grundkörpers 1 nachbildet. Die beiden Schichten 12a und 12b können aus demselben Material und auch einstückig ausgebildet werden oder aber aus unterschiedlichen Materialien bestehen. Die oben beschriebene Gestaltung des Ventilelements 12 ist auch bei einem erfindungsgemäßen Mundstück verwendbar.

Wie beim weiter oben erläuterten Mundstück ist das elastische Ventilelement 12 am Mundstück 1 mit Hilfe einer Klemmeinrichtung 13 befestigt. Gegenüber dem weiter oben erläuterten Mundstück ist jedoch hier, gem. Fig. 2, an der Klemmeinrichtung 13 einstückig eine Ventilelementabdeckung 13a angeformt, die oberhalb des elastischen Ventilelements angeordnet ist und im wesentlichen dieselbe Form wie das elastische Ventilelement 12 besitzt. Aufgabe der Ventilabdeckung 13a ist es, das elastische Ventilelement 12 gegen Berührung und Beschädigung zu schützen. Um dem elastischen Ventilelement 12 zu gestatten, sich während des Ausatmungsvorganges zu bewegen, ist die Ventilabdeckung 13a von dem die Öffnung 14 enthaltenden Wandstück des Bereichs 1b des Mundstücks 1 weggebogen. Der sich dabei ergebende Öffnungswinkel kann in weiten Bereichen frei gewählt werden, solange sichergestellt ist, daß sich das elastische Ventilelement 12 ausreichend bewegen kann, um die Öffnung für die ausgeatmete Luft freizugeben. Auch die oben beschriebene Ventilabdeckung ist bei einem erfindungsgemäßen Mundstück verwendbar.

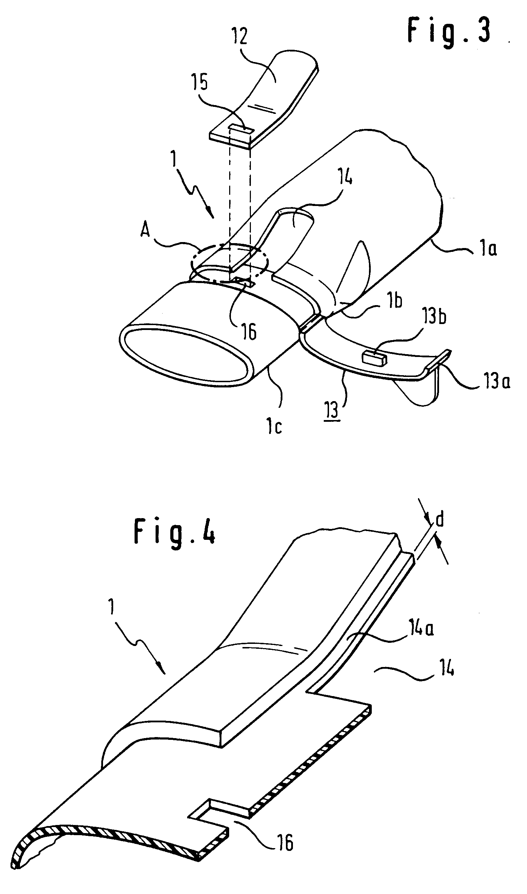

In Fig. 3 ist ein Ausführungsbeispiel eines erfindungsgemäßen Mundstücks gezeigt. Wie bei den zuvor beschriebenen Mundstücken besitzt das erfindungsgemäße Mundstück 1 Bereiche 1a, 1b und 1c. Der Bereich 1a dient zum Befestigen des Mundstücks am Inhalationstherapiegerät und besitzt eine rohrförmige Gestalt. Der Bereich 1b schafft einen Übergang zum ergonomisch geformten Bereich 1c. Im Bereich 1b ist eine Öffnung 14 in einem im wesentlichen ebenen Wandstück des Mundstücks 1 vorgesehen. Ein elastisches Ventilplättchen 12 ist in einer abgehobenen Stellung gezeigt. Im eingebauten Zustand ist das elastische Ventilplättchen 12 so am Mundstück 1 angeordnet, daß eine Arretieröffnung 15, die im elastischen Ventilplättchen 12 vorgesehen ist, über einer Arretieröffnung 16 im Grundkörper 1 des Mundstücks liegt.

Das elastische Ventilplättchen 12 deckt dann die Öffnung 14 des Mundstücks 1 ab, wobei der äußere Rand des beweglichen Teils des Ventilplättchens 1-2 in der Öffnung 14 liegt.

Zur Verdeutlichung ist in Fig. 4 eine vergrößerte Darstellung des in Fig. 3 mit dem Bezugszeichen A gekennzeichneten Teils des erfindungsgemäßen Mundstücks 1 gezeigt. Erkennbar ist, daß die Öffnung 14 am unteren Wandabschnitt eine umlaufenden Randstufe 14a besitzt, die in die Öffnung 14 hineinragt und auf der das elastische Ventilplättchen 12 im Ruhezustand und während der Einatmungsphase aufliegt. Durch diese Ausgestaltung reduziert sich das Totvolumen im Bereich des Mundstückventils auf das durch die mit dem Bezugszeichen d gekennzeichnete Dicke des Randes und die Fläche der Öffnung 14 definierte Volumen.

Die umlaufende Randstufe 14a kann in unmittelbarer Nähe zur Öffnung 14 eine umlaufende Dichtlippe aufweisen, die aus der zum Ventilplättchen gerichteten Oberfläche der Randstufe aufragt; die Dichtlippe ist jedoch in Fig. 4 nicht dargestellt. In diesem Fall liegt das elastische Ventilplättchen auf der Dichtlippe auf.

Das elastische Ventilplättchen 12 besitzt eine Dicke, die nicht größer ist als der verbleibende Wandbereich, an den der Rand des elastischen Ventilplättchens 12 in der Ruhestellung angrenzt. Das Ventilplättchen 12 ragt somit in der Ruhestellung nicht aus der Oberfläche des Mundstücks 1 heraus.

Zur Arretierung ist die Klemmvorrichtung 13 in Form eines Bügels ausgestaltet, der einstöckig mit dem Grundkörper 1 des Mundstücks ausgebildet sein kann. Ein Scharnier gestattet es, daß der Klemmbügel aufgeklappt wird, wie in Fig. 3 gezeigt ist. Das Ventilplättchen 12 wird dann in die Einbaustellung gebracht und der Klemmbügel 13 zugeklappt. Ein Arretiervorsprung 13b auf der dem Ventilplättchen zugewandten Seite des Klemmbügels greift in die Arretieröffnungen 15 und 16 im Ventilplättchen 12 bzw. im Grundkörper 1 ein. Durch geeignete Wahl der Abmessungen ist so ein sicherer Sitz des Ventilplättchens Gewährleistet, das aufgrund des Arretiervorsprungs 13b positioniert und durch den Klemmbügel 13b eingeklemmt ist. Das Ventilplättchen 12 bleibt im Bereich der Öffnung 14 jedoch beweglich, so daß während des Ausatmungsvorgangs Luft aus der Öffnung 14 ausströmen kann. Zum Schutz des elastischen Ventilplättchens gegen Berührungen oder Beschädigungen, insbesondere während des Ausatmungsvorgangs, ist eine Abdeckung 13a am Klemmbügel vorgesehen, die wie beim zweiten Ausführungsbeispiel oberhalb des elastischen Ventilplättchens 12 positioniert ist, wenn der Klemmbügel 13 das Ventilplättchen 12 einklemmt.

## Patentansprüche

1. Mundstück eines Aerosolinhalationstherapiegeräts mit
einem Grundkörper (1) mit im wesentlichen rohrförmiger Gestalt, der zumindest einen im wesentlichen ebenen Wandabschnitt aufweist, in dem eine Öffnung (14) mit einer umlaufenden Randstufe (14a), die sich in die Öffnung (14) hinein erstreckt, ausgebildet ist, und
einem Rückschlagventil (11) zum Abdichten der Öffnung (14) während eines Einatmungsvorgangs durch das Mundstück und zum Abführen von Atemluft durch die Öffnung (14) während eines in das Mundstück gerichteten Ausatmungsvorgangs, das ein flaches, elastisches Ventilelement umfaßt,
- das an dem Grundkörper (1) in dem Bereich des ebenen Wandabschnitts derart befestigt ist,
-- daß es in einer Ruhestellung aufgrund der wirkenden elastischen Kräfte von außen am Grundkörper (1) anliegt und die Öffnung (14) verschließt, und
-- daß es zur Freigabe der Öffnung (14) von dem Grundkörper weg teilweise abhebbar ist,
- dessen Abmessungen und Form zum Verschließen der Öffnung (14) an diese angepaßt sind, und
- das auf der Randstufe (14a) in der Ruhestellung derart aufliegt, daß das flache, elastische Ventilelement (12) im wesentlichen in der Öffnung (14) angeordnet ist.

2. Mundstück nach Anspruch 1,
dadurch **gekennzeichnet**, daß
der Grundkörper (1) einen Anschlußbereich (1a) für den Anschluß des Mundstücks an das Inhalationstherapiegerät, einen ergonomisch geformten Bereich (1c) zur Aufnahme in den Mund und einen Übergangsbereich (1b) zur Überführung der Form des Anschlußbereichs (1a) in der Form des ergonomisch geformten Bereichs (1c) aufweist, und daß der im wesentlichen ebene Wandabschnitt, in dem die Öffnung (14) ausgebildet ist, im Übergangsbereich (1b) angeordnet ist.

3. Mundstück nach Anspruch 1,
dadurch **gekennzeichnet**, daß
das flache elastische Ventilelement (12) mittels einer Klemmeinrichtung (13) aussen am Grundkörper (1) gehaltert ist.

4. Mundstück nach Anspruch 1,
dadurch **gekennzeichnet**, daß
an die Klemmeinrichtung (13) einstückig eine Ventilelementabdeckung (13a) angeformt ist, die zum Schutz des flachen, elastischen Ventilelements aussen am Grundkörper (1) über dem Ventilelement (12) angeordnet ist.

5. Mundstück nach einem der Ansprüche 1 bis 4,
dadurch **gekennzeichnet**, daß
die Randstufe (14a) mit einer umlaufenden Dichtlippe ausgestattet ist.

6. Mundstück nach einem der Ansprüche 3 bis 5,
dadurch **gekennzeichnet**, daß
das flache elastische Ventilelement (12) eine Arretieröffnung (15) aufweist, daß der Grundkörper (1) eine Arretieröffnung (16) aufweist und daß die Klemmeinrichtung (13) aus einer geöffneten Stellung in eine das Ventilelement einklemmende Stellung klappbar ist und einen Arretiervorsprung (13b) aufweist, der in der das Ventilelement einklemmenden Stellung der Klemmeinrichtung (13) in das zueinander ausgerichteten Arretieröffnungen (15, 16) des Ventilelements (12) und des Grundkörpers (1) eingreift.

7. Mundstück nach einem der Ansprüche 1 bis 6,
dadurch **gekennzeichnet**, daß
das flache, elastische Ventilelement (12) zwei Schichten (12a, 12b) aufweist, von denen die erste Schicht (12a) aussen am Grundkörper (1) anliegt und die zweite Schicht (12b) in der Ruhestellung des Ventilelements (12) derart in der Öffnung (14) angeordnet ist, daß die zum Innenraum des Grundkörpers (1) gerichtete Oberfläche der zweiten Schicht (12b) die Innenwand des Grundkörpers (1) im Bereich der Öffnung (14) nahezu vollständig fortsetzt.

8. Mundstück nach Anspruch 7,
dadurch **gekennzeichnet**, daß
das Ventilelement (12) einstückig oder aus zwei getrennten Schichten (12a, 12b) aufgebaut ist, die aus gleichen oder unterschiedlichen Materialien bestehen.

9. Mundstück nach einem der Ansprüche 1 bis 8,
dadurch **gekennzeichnet**, daß
das flache, elastische Ventilelement (12) aus einem Kunststoff oder Gummi hergestellt ist.

## Claims

1. Mouthpiece of an aerosol inhalation therapy unit having a basic body (1) of essentially tubular shape with an essentially flat wall section in which is configured an opening (14) with a surrounding edge section (14a) which extends into the opening (14) and having a non-return valve (11) for sealing the opening (14) during an inhalation procedure through the mouthpiece and for removing exhaled air through the opening (14) during an exhalation procedure directed into the mouthpiece, which non-return valve (11) includes a flat, elastic valve element,
- which is fastened to the basic body (1) in the region of the flat wall section in such a way
• that, in an at-rest position, it is in contact with the basic body (1) from the outside because of the elastic forces operating and thus closes the opening (14), and
• that it can be partially lifted from the basic body in order to free the opening (14),
- which valve element is matched in dimensions and shape to the opening (14) in order to close the latter, and
- which valve element, in the at-rest position, is in contact with the edge section (14a) in such a way that the flat, elastic valve element (12) is essentially arranged within the opening (14).

2. Mouthpiece according to Claim 1, characterised in that the basic body (1) has a connection region (1a) for connecting together the mouthpiece onto the inhalation therapy unit, an ergonomically shaped region (1c) (to be taken into the mouth) and a transition region (1b) for converting the shape of the connection region (1a) into the shape of the ergonomically shaped region (1c), and in that the essentially flat wall section, in which the opening (14) is configured, is arranged in the transition region (1^{b}).

3. Mouthpiece according to Claim 1, characterised in that the flat elastic valve element (12) is held externally on the basic body (1) by means of a clamping device (13).

4. Mouthpiece according to Claim 1, characterised in that there is a valve element cover (13a) integrally formed on the clamping device (13), which valve element cover (13a) is arranged externally on the basic body (1) above the valve element (12) in order to protect the flat, elastic valve element.

5. Mouthpiece according to one of Claims 1 to 4, characterised in that the edge section (14a) is provided with a circumferential sealing lip.

6. Mouthpiece according to one of Claims 3 to 5, characterised in that the flat elastic valve element (12) has a locking opening (15), in that the basic body (1) has a locking opening (16) and in that the clamping device (13) can be folded from an open position into a position clamping the valve element and has a locking projection (13b) which, in the position of the clamping device (13) which clamps the valve element, engages in the mutually aligned locking openings (15, 16) of the valve element (12) and the basic body (1).

7. Mouthpiece according to one of Claims 1 to 6, characterised in that the flat, elastic valve element (12) has two layers (12a, 12b) of which, in the at-rest position of the valve element (12), the first layer (12a) is in contact on the outside of the basic body (1) and the second layer (12b) is arranged in the opening (14) in such a way that the surface of the second layer (12b) which is directed towards the internal space of the basic body (1) provides an almost complete continuation of the inner wall of the basic body (1) in the region of the opening (14).

8. Mouthpiece according to Claim 7, characterised in that the valve element (12) is constructed in one piece or is constructed of two separated layers (12a, 12b) which consist of the same or different materials.

9. Mouthpiece according to one of Claims 1 to 8, characterised in that the flat, elastic valve element (12) is manufactured from a plastic or rubber.

## Revendications

1. Embout buccal pour appareil d'aérocol de thérapie par inhalation, comportant
un corps de base (1) ayant une forme sensiblement tubulaire, présentant au moins un tronçon de paroi sensiblement plan, dans lequel est ménagée ouverture (14) avec un étagement de bordure (14a) de pourtour qui s'étend dans l'ouverture (14), et
un clapet anti-retour (11), destiné à isoler de façon étanche l'ouverture (14) pendant le processus d'inspiration-inhalation effectué au moyen de l'embout buccal et à assurer l'évacuation de l'air de la respiration par l'ouverture (14) pendant un processus d'expiration dirigé dans l'embout buccal, comprenant un élément soupape, élastique, plat,
- qui est fixé sur le corps de base (1), dans la zone du tronçon de paroi plan, de manière que,
-- dans une position de repos, il appuie de l'extérieur sur le corps de base (1), du fait des efforts élastiques en action, et ferme l'ouverture (14), et
-- il puisse être soulevé partiellement du corps de base, pour libérer l'ouverture (14),
- dont les dimensions et la forme sont adaptées à l'ouverture (14) pour fermer cette dernière, et
- qui, dans la position de repos, repose sur l'étagement de bordure (14a), de manière que l'élément soupape (12) élastique plat soit disposé sensiblement dans l'ouverture (14).

2. Embout buccal selon la revendication 1,
caractérisé en ce que le corps de base (1) présente une zone de raccordement (1a) pour le raccordement de l'embout buccal à l'appareil de thérapie par inhalation, une zone (1c) à forme ergonomique, destinée à être reçue dans la bouche, et une zone de transition (1b) destinée à assurer le passage de la forme de la zone de raccordement (1a) en la forme de la zone forme ergonomique (1c), et en ce que le tronçon de paroi sensiblement plan, dans lequel l'ouverture (14) est réalisée, est disposé dans la zone de transition (1^{b}).

3. Embout buccal selon la revendication 1,
caractérisé en ce que l'élément soupape (12) élastique plat est maintenu extérieurement sur le corps de base (1) au moyen d'un dispositif de serrage (13).

4. Dispositif selon la revendication 1,
caractérisé en ce que, sur le dispositif de serrage (13) est formé d'un seul tenant, d'une seule pièce, un recouvrement d'élément soupape (13a) qui est disposé extérieurement sur le corps de base (1), sur l'élément soupape (12) dans le but de protéger l'élément soupape élastique plat.

5. Embout buccal selon l'une des revendications 1 à 4, caractérisé en ce que l'étagement de bordure (14a) est doté d'une lèvre d'étanchéité faisant le pourtour.

6. Embout buccal selon l'une des revendications 3 à 5, caractérisé en ce que l'élément soupape (12) élastique plat présente une ouverture de blocage (15) en ce que le corps de base (1) présente une ouverture de blocage (16) et en ce que le dispositif de serrage (13) peut être rabattu, d'une position ouverte en une position enserrant l'élément soupape, et présente une saillie de blocage (13b) qui, dans la position enserrant l'élément soupape du dispositif de serrage (13), s'engage dans les ouvertures de blocage (15, 16) orientées l'une par rapport à l'autre, de l'élément soupape (12) et du corps de base (1).

7. Embout buccal selon l'une des revendications 1 à 6, caractérisé en ce que l'élément soupape (12) élastique plat présente deux couches (12a, 12b) dont la première couche (12a) appuie extérieurement sur le corps de base (1) et la deuxième couche (12b) en position de repos de l'élément soupape (12), est disposée dans l'ouverture (14) de manière que la surface, tournée vers l'espace intérieur du corps de base (1), de la deuxième couche (12b) continue à peu près complètement la paroi intérieure du corps de base (1) dans la zone de l'ouverture (14).

8. Embout buccal selon la revendication 7, caractérisé en ce que l'élément soupape (12) est constitué d'une seule pièce ou de deux couches (12a, 12b) séparées, qui sont constituées de matériaux identiques ou différents.

9. Embout buccal selon l'une des revendications 1 à 8, caractérisé en ce que l'élément soupape (12) élastique plat est fabriqué en matière plastique ou en caoutchouc.
